# EUROPÄISCHE PATENTANMELDUNG

(11) **EP 0 761 690 A1**
(43) Veröffentlichungstag der Anmeldung: **12.03.1997**
(21) Anmeldenummer: 96112211.6
(22) Anmeldetag: 29.07.1996
(51) Int. Cl.: C08B 13/00, C08B 31/04

(54) **Maleinsäureadditionsproduktgruppen enthaltende, thermoplastische und biologisch abbaubare Polysaccharidester/-etherester**

(30) Priorität: 10.08.1995 DE 19529409
(71) Anmelder: WOLFF WALSRODE AG, D-29655 Walsrode (DE)
(72) Erfinder: Kalbe, Jochen, Dr., 45219 Essen (DE); Koch, Reinhard, Dr., 51065 Köln (DE); Müller, Hanns Peter, Dr., 51519 Odenthal (DE); Engelhardt, Jürgen, Dr., 29683 Fallingbostel (DE); Koch, Wolfgang, Dr., 29699 Bomlitz (DE); Müller, Volkhard, Dr., 29699 Bomlitz (DE)
(74) Vertreter: Braun, Rolf, Dr.

(57) **Zusammenfassung**

Die vorliegende Erfindung betrifft thermoplastische und biologisch abbaubare Polysaccharidester/Polysaccharidetherester, die dadurch gekennzeichnet sind, daß sie sich durch die allgemeine Struktur 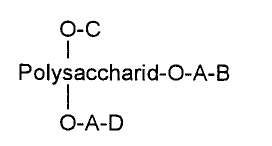 beschreiben lassen, wobei Polysaccharid-O die substituierten OH-Gruppen einer polymeren Saccharideinheit repräsentiert und in der A eine lineare Polyetherkette folgender Struktur ist

A = (E-O)n

in der E eine lineare aliphatische oder aromatische verzweigte oder unverzweigte Kette mit 2 bis 11 C-Atomen bedeutet, n eine ganze Zahl gleich oder größer als 0 ist und B sowie D ein Maleinsäureadditionsprodukt der folgenden Struktur ist 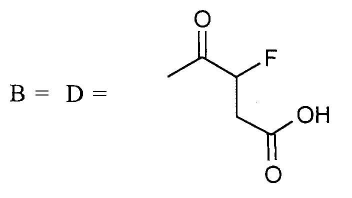 in der F ein aliphatisches, gesättigtes, ein- oder mehrfach ungesättigtes Kohlenstoffgerüst ist, das gegebenenfalls mit weiteren Substituenten versehen sein kann und wobei C ein Wasserstoff, ein oder mehrere Substituenten aus der Gruppe Hydroxyethyl, Hydroxypropyl, Hydroxybutyl, Methyl, Ethyl, Benzyl, Dihydroxypropyl, Carboxyalkyl, Sulfoalkyl oder Cyanoethyl sein kann.

## Beschreibung

Die vorliegende Erfindung betrifft neue thermoplastische und biologisch abbaubare Polysaccharidester und Polysaccharidetherester, wie z.B. Stärkeester, Stärkeetherester und Celluloseetherester sowie die Herstellung solcher Polysaccharidester/Polysaccharidetherester von Maleinsäureadditionsprodukten wie z.B. Alkyl- und/oder Alkenylbernsteinsäure, des weiteren gemischte Ester von Polysacchariden/Polysaccharidethern, die als Esterkomponente Maleinsäureadditionsprodukte und weitere Dicarbonsäure- oder Monocarbonsäuregruppen enthalten.

In der Literatur beschriebene Celluloseetherester bestehen aus Celluloseethern, die in verschiedenen Verfahren mit Carbonsäureanhydriden zu deren Monoestern umgesetzt werden. [siehe C.J. Malm, Analytical Chemistry, 25(2), 1953, 245-249; C.J. Malm, Industrial and Engineering Chemistry, 49(1), 1957, 84-88; EP 0 219 426 (06.10.86), DOS 2 140 996 (16.08.71)].

Auch verschiedene Arten gemischter Monocarbonsäure-, Dicarbonsäureester von Cellulose oder Celluloseethern, wie z.B. Celluloseacetatphthalat (Veröffentlichung Nr. ZFD-101 B der Firma Eastman Chemical Produkts Inc., USA, 1975 und G.T. Luce in Pharmaceutical Technology, Juni 1977), Celluloseacetatsuccinat [J. of Pharm. Sci., 51, 1962, 484; Chem. Abstr. 76, 1972, 125, Ref. 129 046 t; Chem. Abstr. 81, 1974, 143, Ref. 154 839 q] und Hydroxypropylcelluloseacetatsuccinat [EP 0 219 426 (06.10.86)] sind lange bekannt.

Je nach Anforderung können dabei durch Variation des Etherderivats, z.B. Methyl-, Ethyl-, Hydroxyethyl-, Hydroxypropyl-, Propyl-, Butylcellulose, oder auch Mischethertypen wie Methylhydroxyethyl-, oder Methylhydroxypropylcellulose oder durch die Wahl verschiedener Dicarbonsäureanhydride wie z.B. Phthalsäure, Tetrahydrophthalsäure, Hexahydrophthalsäure, Bernsteinsäure oder durch variierenden Substitutionsgrad dieser Substitutienten, Eigenschaftsprofile eingestellt werden [siehe DOS 214 0996 (16.08.71), EP 0 219 426 (06.10.86)].

Bei dieser Produktklasse handelt es sich um aus organischen Lösungsmitteln oder aus Wasser applizierbare Lacksysteme zur Tablettenverkapselung oder um Lichthofschutzschichten.

Die biologische Abbaubarkeit eines Polysaccharidderivates ist abhängig von der Höhe des Substitutionsgrades jeder Saccharideinheit [siehe J.G. Batelaan in The Handbook of Environmental Chemistry, Volume 3, Part F, Ed. O. Hutzinger, Springer-Verlag, 1992, 229-336, M.G. Wirick, Journal of Polymer Science, Part A-1, 6(1968), 1705-1718]. So sind alle technisch verfügbaren Cellulosederivate nur mit durchschnittlichen Substitutionsgraden kleiner als 1,0 biologisch leicht abbaubar. Thermoplastizität ist dagegen bei bekannten Derivaten wie z.B. Celluloseacetat, erst mit Substitutionsgraden größer als 2,5 zu erzielen (T. Eicher, in Ullmanns Encyklopädie der technischen Chemie, 4. Auflage, 9, 1975, 227-246).

Die in der Regel gut biologisch abbaubaren, wenig modifizierten Polysaccharide können demnach technologischen Anforderungen wie z.B. Extrusionsfähigkeit auf herkömmlichen Extrusionsanlagen, aber auch Anforderungen wie Wasserdampffestigkeit und Wasserdichtigkeit nicht genügen und weisen keine mit Standardkunststoffen vergleichbaren mechanischen Eigenschaften auf [K. Dormann, Zuckerind. 116(7), 191, 620-623].

So existieren thermoplastische und damit auch zu Formteilen, wie z.B. Folien, extrudierbare Cellulosederivate, die gleichzeitig vollständig biologisch abbaubar sind, bis auf die in den Patentanmeldungen DE 4 317 231 und EP 0584 677 beschriebenen Substanzen nicht.

Stärkeester sind bei Substitutionsgraden größer 1 zwar noch leicht biologisch abbaubar, sie können jedoch nur mühsam und nach Zugabe beträchtlicher Weichmachermengen thermoplastisch verarbeitet werden (DE 43 26 118 A1).

Aufgabe der vorliegenden Erfindung ist demnach die Synthese neuartiger thermoplastischer, extrudierbarer und biologisch abbaubaren Polysaccharidester/-etherester.

Erfindungsgemäß gelingt dies durch Veresterung von Polysacchariden oder Polysaccharidethern mit Maleinsäureanhydridadditionsprodukten. Die bevorzugten Maleinsäureanhydridadditionsprodukte sind Alkanyl- und/oder Alkenylbernsteinsäureanhydride, wobei der gegebenenfalls eine oder mehrere Doppelbindungen enthaltende Alkylrest vorzugsweise 8 bis 18 C-Atome aufweist sowie Maleinsäureanhydridadditionsprodukte an Isobutylen und Polyenfettsäurederivate. Bei diesen Additionsprodukten handelt es sich um bekannte Verbindungen, die durch indirekte substituierende Addition von alpha-Olefinen an Maleinsäureanhydrid (En-Synthese) dargestellt werden.

Weiterhin wird die Aufgabe gelöst durch die Synthese neuartiger gemischter Ester aus Polysacchariden/Polysaccharideethern, Maleinsäureanhydridadditionsprodukten und weiteren Dicarbonsäureanhydriden wie Phthalsäureanhydrid, Tetrahydrophthalsäureanhydrid, Hexahydrophthalsäureanhydrid oder Maleinsäureanhydrid sowie durch die gemischten Polysaccharidester/-etherester von Maleinsäureadditionsprodukten und Monocarbonsäuren wie Essigsäure, Propionsäure oder Buttersäure.

Gegenstand der vorliegenden Erfindung sind somit thermoplastische und biologisch abbaubare Polysaccharidderivate aus Polysacchariden oder Polysaccharidethern, die mit Maleinsäureanhydridadditionsprodukten umgesetzt werden wie auch Polysaccharidester/-etherester, die neben Maleinsäureadditionsprodukten weitere Dicarbonsäure- oder Monocarbonsäuregruppen enthalten.

Die Erfindungsgemäß herzustellenden Polysaccharidetherester lassen sich durch die allgemeine Struktur 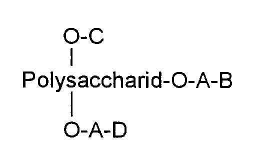 beschreiben, wobei Polysaccharid-O die substituierten OH-Gruppen einer polymeren Saccharideinheit repräsentiert und in der A eine lineare Polyetherkette folgender Struktur ist

A = -(E-O)n

in der E eine lineare aliphatische oder aromatische verzweigte oder unverzweigte Kette mit 2 bis 11 C-Atomen bedeutet, n eine ganze Zahl gleich oder größer als 0 ist und B ein Maleinsäureanhydridadditionsprodukt der folgenden Struktur ist 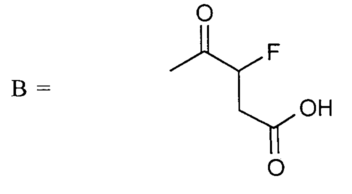 in der F ein aliphatisches, gesättigtes, ein- oder mehrfach ungesättigtes Kohlenstoffgerüst ist, das gegebenenfalls mit weiteren Substituenten versehen sein kann und wobei C ein Wasserstoff, ein oder mehrere Substituenten aus der Gruppe Hydroxyethyl, Hydroxypropyl, Hydroxybutyl, Methyl, Ethyl, Benzyl, Dihydroxypropyl, Carboxyalkyl, Sulfoalkyl oder Cyanoethyl sein kann.

D ist entweder gleich B oder eine Carbonsäure der Struktur 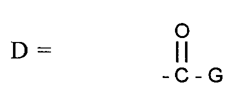 wobei G ein aliphatisches C-1 bis C-3 Kohlenstoffgerüst ist, das gegebenenfalls mit weiteren Substituenten versehen sein kann oder G entspricht der Struktur 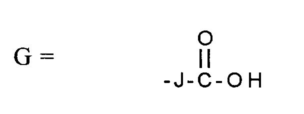 in der J ein aromatisches oder aliphatisches Kohlenstoffgerüst, das gegebenenfalls mit weiteren Substitutienten versehen sein kann ist.

Die erfindungsgemäßen Produkte sind mit bekannten wasserlöslichen Alkanyl- und/oder Alkenylbernsteinsäureestergruppierungen enthaltenden Celluloseethern und Stärken (EP 024 025 B1 und US 4 035 235) in keiner Weise vergleichbar, da sie mit höheren Substitutionsgraden (DS) der Estergruppierungen versehen sind und somit völlig andere Eigenschaftsprofile aufweisen. Sie sind hydrophob, organolöslich sowie überraschenderweise thermoplastisch und biologisch abbaubar.

Zur Synthese dieser Polysaccharidester/-etherester von Maleinsäureadditionsprodukten insbesondere von Alkenyl- und/oder Alkanylbernsteinsäure mit aliphatischen C-8 bis C-18 Seitenketten, wird das Polysaccharidderivat in einem organischen Lösungsmittel suspendiert und mit dem Katalysator versetzt. Anschließend gibt man das Maleinsäureanhydridadditionsprodukt zu. Als Katalysatoren eignen sich basische Verbindungen wie z.B. aliphatische, cycloaliphatische und aromatische Amine sowie basische anorganische Salze. Die so entstanden Polysaccharidester/-etherester sind im Suspensions- bzw. Lösungsmittel vollständig gelöst und können durch Abdestillation des Lösungsmittels oder Fällung in Nichtlösemitteln wie z.B. Wasser isoliert werden.

Die Reaktionstemperatur der Veresterung liegt zwischen 20 und 150°C, bevorzugt zwischen 80 und 120°C, die Reaktionszeit zwischen 1 und 10 Stunden, bevorzugt zwischen 2 und 4 Stunden.

Die gemischten Ester von Polysacchariden/Polysaccharidestern mit Maleinsäureadditionsprodukten und weiteren Dicarbonsäuren sind auf diesem Wege ebenfalls zugänglich. So lassen sich ohne die Thermoplastizität und die biologische Abbaubarkeit der erfindungsgemäßen Produkte zu beeinträchtigen bis zu 96,5 Mol % Maleinsäureanhydridadditionsprodukt durch andere Polycarbonsäureanhydride substituieren. Geeignete Dicarbonsäureanhydride sind Phthalsäureanhydrid, Tetrahydrophthalsäureanhydrid, Maleinsäureanhydrid und Bernsteinsäureanydrid. Weiterhin eignen sich Isatosäureanhydrid, Trimellitsäureanhydrid und Pyromellitsäureanhydrid.

Auch im Falle der Polysaccharidester/Polysaccharidetherester von C-2 bis C-4 Monocarbonsäuren, insbesondere von Essigsäure, Propionsäure und Buttersäure, läßt sich thermoplastische Verarbeitbarkeit bei gleichzietiger biologischer Abbaubarkeit erreichen, wenn zusätzlich Maleinsäureadditionsproduktgruppen eingeführt werden.

Geeignete Polysaccharide sind beispielsweise native und lösliche Stärke beliebiger Provinienz, Amylose, Amylopektin, Alginat, Carraghenan, Chitin, Chitosan, Dextran, Glycogen, Guar, Johannisbrotkernmehl, Laevan, Pektin, Pollulan, Tamarindenkernmehl, Xanthan und Xylan. Werden Polysaccharidether eingesetzt, eignen sich Hydroxyethylstärke, Hydroxypropylstärke oder Hydroxypropylguar und insbesondere Celluloseether wie Methylcellulose, Ethylcellulose, Benzylcellulose, Hydroxyethylcellulose, Dihydroxypropylcellulose, Hydroxybutylcellulose, Methylhydroxyethylcellulose, Methylhydroxypropylcellulose, Methylhydroxybutylcellulose, Ethylhydroxypropylcellulose, Ethylhydroxyethylcellulose, Carboxyalkylcellulose, Sulfoalkylcellulose oder Cyanoethylcellulose und deren Mischether mit durchschnittlichen Substitutionsgraden (DS) kleiner/gleich 1,5, bevorzugt kleiner 1.

Als Suspensions- bzw. Lösungsmittel eignenen sich Ketone, Ether und cyclische Ether, Acetale, Kohlenwasserstoffe und polare aprotische Verbindungen wie Dimethylsulfoxid, Dimethylformamid, Dimethylacetamid, Dioxan, Tetrahydrofuran, N-Methylmorpholin, N-Methylpyrolidon, Dimethoxymethan, Dimethylether, Diethylenglycoldimethylether des weiteren protische Lösungsmittel wie Isopropanol, tert.-Butanol, Essigsäure und in besonderen Fällen auch Wasser.

Die als Katalysatoren eingesetzten Amine sind insbesondere Trimethylamin, Triethylamin, Tributylamin, Tetramethylendiamin, Pyridin, N,N-Dimethylcyclohexyldiamin, N,N-Dimethylbenzylamin, 4-Pyrrilidinopyridin, Permethyldiethylentetramin, 1,4-Diazabicyclo[2.2.2]octan, 1,8-Diazabicyclo[5.4.0]undec-7-en, 1,5-Diazabicyclo[4.3.0]non-5-en oder beliebige Mischungen aus ihnen.

Dienen basische, anorganische Salze als Katalysatoren, eignen sich Natriumacetat, Natriumcarbonat, Kaliumacetat, Kaliumcarbonat und Lithiumcarbonat.

Im Falle der Veresterung von Celluloseethern mit Maleinsäureanhydridadditionsprodukten und Monocarbonsäureanhydriden kann die Reaktion auch säurekatalysiert durchgeführt werden. Dazu wird der Celluloseether in einer aliphatischen C-2 bis C-4 Monocarbonsäure, insbesondere in Essigsäure suspendiert und mit einem sauren Katalysator versetzt. Nach Quellung des Eduktes erfolgt zunächst die Zugabe von C-2 bis C-4 Monocarbonsäureanhydrid wie z.B. Acetanhydrid, Propionsäureanhydrid oder Buttersäureanhydrid, gefolgt von der Zugabe des Maleinsäureadditionsprodukts wie z.B. Alkanyl- und/oder Alkenylbernsteinsäureanhydrid. Die Reaktionszeit beträgt 1 bis 10 Stunden, insbesondere 2 bis 4 Stunden, die Reaktionstemperatur 40 bis 80°C insbesondere 40 bis 50°C, um einen zu weitgehenden Abbau der Polysaccharidketten zu vermeiden. Zur Aufarbeitung wird die homogene Reaktionslösung mit wäßriger NaOH versetzt und das Lösungsmittel entweder destillativ oder durch Fällung in Wasser entfernt.

Als Katalysatoren eignen sich starke Mineralsäuren wie z.B. conc. Schwefelsäure oder Perchlorsäure.

Geeignete Lösungsmittel/Acylierungsmittel-Kombinationen sind Essigsäure/Acetanhydrid, Propionsäure/Propionsäureanhydrid.

Das Verfahren zur Darstellung dieser Derivate entspricht der lange bekannten Synthese von Celluloseacetat (L.J. Tanghe, L.B. Genung, J.W. Mench, Methods in Carbohydrate Chemistry III, 1963, 193-198), mit dem Vorteil, daß beim Einsatz von Celluloseethern als Edukte auch partiell substituierte Cellulosederivate direkt zugänglich werden. Es zeichnet sich weiterhin durch eine wenig aufwendige Durchführung aus, da das in Polysacchariddderivaten naturgemäß vorliegende Wasser mit den Monocarbonsäureanhydriden zu den korrespondierenden Monocarbonsäuren abreagiert, die gleichzeitig das Reaktionsmedium bilden. Nebenprodukte sind somit destillativ entfernbar, was die weitere Reinigung des Produkts wie auch die des Lösungsmittels überflüssig macht.

Durch Variation des zugrundeliegenden Polysaccharidderivates, der Etherkomponente und der Estergruppierung sowie durch die Wahl der Substitutionsgrade kann man so thermoplastische und biologisch abbaubare Polysaccharidderivate mit Schmelzpunkten zwischen 80 und 190°C erhalten, die mit den herkömmlichen Verarbeitungstechniken für Thermoplaste wie Extrusion, Spritzguß oder Blasformen verarbeitbar sind.

Die erfindungsgemäßen Produkte zeigen bemerkenswerte und in keiner Weise vorhersehbare Eigenschaften: Celluloseacetate werden erst ab einem DS > 2,5 thermoplastisch und erfordern auch nach Zugabe beträchtlicher Weichmachermengen noch Verarbeitungstemperaturen > 200°C [F. Müller, Ch. Leuschke in Becker/Braun (Ed.), Kunststoff-Handbuch Band 3/1: Polycarbonate, Polyacetale, Celluloseester, 1992, 437-445 ]. Hydroxyalkylcelluloseacetatealkenylsuccinate mit ähnlichem Substitutionsgrad dagegen erweichen ohne externe Weichmachung schon bei Temperaturen unter 100°C. Durch die Art des Celluloseethers und der Esterkomponenten, die Variation der Substitutionshöhe und das Verhältnis Alkenylbernsteinsäureanhydrid/Dicarbonsäure oder C-2 bis C-4-Monocarbonsäure sind Polysaccharidester/Polysaccharidetherester mit niedrigen Substitutionsgraden zugänglich, die dennoch für gängige Thermoplasten typische Erweichungspunkte zwischen 150 und 190°C haben.

Die erfindungsgemäßen Polysaccharidderivate sind geeignet zur Herstellung von z.B. biologisch abbaubaren Folien, Fasern, Töpfen Flaschen und anderen Formkörpern sowie als Matrixmaterial für Formulierungen für die verzögerte Freisetzung von Wirkstoffen (z.B. Pheromone, Dünger, Fungizide, Insektizide, Herbizide oder Nematozide).

Sie können in ihren Eigenschaften durch die Herstellung von Blends beliebiger Zusammensetzung mit anderen biologisch abbaubaren Komponenten wie z.B. Stärke, Cellulose, Polylactid, Glycolid, Polyhydroxybuttersäure Polyhydroxyvaleriansäure, Polycaprolacton, Polyesteramiden oder Polyesterurethanen variiert werden. Die Modifizierung mit Hilfsmitteln wie z.B. Weichmachern, Antioxidantien, Witterungsstabilisatoren, Flammschutzmitteln, Farbstoffen oder Pigmenten ist möglich. Weiterhin gelingt die Herstellung von kompostierbaren anisotropen Faserverbundwerkstoffen durch Einbringen von natürlichen Fasern wie Flachs, Cellulose, Ramie oder Hanf.

Die beschriebenen Polysaccharidderivate sind in organischen Lösungsmitteln wie z.B. DMSO, DMAc, Dioxan, THF oder Aceton löslich.

Gegenüber den bekannten Polysacchariddicarbonsäurehalbestern zeichnen sich die erfindungsgemäßen Polysaccharidderivate durch eine Reihe von Vorteilen aus:
- Sie sind in einfachen Rührautoklaven des Stands der Technik zu synthetisieren.
- Die Derivatisierung kann in organischen Lösungsmitteln durchgeführt werden, wobei das entstehende Polysaccharidderivat gelöst wird.
- Die Derivate lassen sich als freifließende thermoplastische Pulver mit weit einstellbarem Schmelzbereich gewinnen.
- Die freifließenden Pulver sind auf herkömmlichen Extrudern thermoplastisch verarbeitbar.
- Es können in wirtschaftlicher Weise Folien und Formteile in hoher Qualität hergestellt werden.
- Die Folien und Formkörper sind geruchsfrei, wasserfest und erfüllen alle Anforderungen an das Leistungsprofil herkömmlicher Materialien.
- Die resultierenden Folien und Formkörper sind biologisch abbaubar.

Die neuen erfindungsgemäßen Polysaccharidether/-etherester eignen sich zur Herstellung von Formteilen wie z.B. Flaschen, Blumentöpfen, Einmalbesteck und -geschirr, Golftees, Folien zur Verpackung von Lebensmitteln und Bioabfällen, Mulchfolien, Babywindeln usw.. Weiterhin sind sie geeignet zur Beschichtung von Flächengebilden wie z.B. Papier, Fließen, Geweben, Gewirken oder anderen Substraten oder auch zur Herstellung von Fasern Blends und Laminaten. Entsprechende Materialien sind auch z.B. dem Papierrecycling zugänglich.

Die Eigenschaft der biologischen Abbaubarkeit ist für die Produkte der Beispiele 1 bis 7 gegeben und wird dabei wie folgt untersucht:

Je 2,5 g der gemahlenen Probe wurde mit 20 g durchgerottetem Kompost und 1 ml Wasser vermischt. Die Mischung wurde in eine 1-l-Flasche mit Spezialverschluß gegeben und der Luftüberstand durch reinen Sauerstoff ersetzt. Nach 7, 14 und 28 Tagen erfolgte die CO2-Bestimmung in der Gasphase. Nach jeder CO2-Bestimmung wurde die Gasphase über der Prüfsubstanz durch reinen Sauerstoff erneuert. Die Inkubationstemperatur betrug 37°C. Parallel wurde die CO2-Entwicklung von Positivkontrollen (Zellstoff) sowie 0-Proben (ohne Zusatz weiterer biologisch abbaubarer Komponenten) bestimmt.

Der Gegenstand der vorliegenden Erfindung soll anhand der vorliegenden Beispiele noch näher erläutert werden.

### Beispiel 1

Eine Hydroxypropylcellulose (100g / 0,46 Mol) mit molarem Substitutionsgrad (MS) 0,93 wird in 900 g *tert.*-Butanol suspendiert, mit 1,2 g 1,8-Diazabicyclo[5.4.0]undec-7-en (DBU) versetzt und auf 80°C geheizt. Nach Zugabe von 233 g (0,88 Mol) 2-Dodecenylbernsteinsäureanhydrid erhöht man die Temperatur auf 120°C, rührt 3 h und bringt die resultierende pastöse Masse in Wasser ein. Das ausfallende Produkt wird gewaschen und getrocknet. Es weist einen Erweichungsbereich von 130-140°C auf.

### Beispiel 2

Eine Hydroxyethylcellulose (75g / 0,39 Mol) mit MS 0,68 wird in 900 g Dioxan suspendiert, mit 2,5 g DBU versetzt und 2 h auf 80°C geheizt. Nach der Zugabe von 86 g (0,59 Mol) Phthalsäureanhydrid und 52 g (0,2 Mol) 2-Dodecenylbernsteinsäureanhydrid erhöht man die Temperatur für 3 h auf 120°C, kühlt ab, fällt aus Wasser und isoliert das Produkt, welches sich ab 210°C mechanisch verformen läßt.

### Beispiel 3

75 g (0,39 Mol) einer Hydroxyethylcellulose mit MS 0,68 wird in 2000 g Eisessig suspendiert, 2 h bei Raumtemperatur gerührt und mit 60 g (0,59 Mol) Acetanhydrid versetzt, das 1 ml conc. Schwefelsäure enthält. Man rührt das Reaktionsgemisch 1 h bei 50°C, versetzt anschließend mit 65 g (0,29 Mol) 2-Nonenylbernsteinsäureanhydrid und gibt nach weiteren 2 h 4 ml 50proz. wäßrige Natriumhydroxidlösung zu. Eindampfen des Lösungsmittels, waschen mit Wasser und trocknen liefert ein Produkt vom Schmelzbereich 170-180°C.

### Beispiel 4

75 g (0,35 Mol) einer Hydroxypropylcellulose mit MS 0,84 wird in 2000 g Eisessig suspendiert, 2 h bei Raumtemperatur gerührt und mit 55 g (0,54 Mol) Acetanhydrid versetzt, das 1 ml conc. Schwefelsäure enthält. Man rührt das Reaktionsgemisch 1 h bei 50°C, versetzt anschließend mit 72 g (0,27 Mol) 2-Dodecenylbernsteinsäureanhydrid und gibt nach weiteren 2 h 4 ml 50proz. wäßrige Natriumhydroxidlösung zu. Eindampfen des Lösungsmittels, waschen mit Wasser und trocknen liefert ein thermoplastisches Produkt.
- MFR 160°C/ 2.16 Kg: 1.35 g in 10 min: O₂-Permeation: 310 cm³/m² d bar (DIN 53380)
- E-Modul: 153/121 M Pa (DIN 53457): H₂O-Permeation 23°C/85%: 19 g/m² d (DIN 53122)
- Reißfestigkeit: 7.1 M Pa (DIN 53455): CO2-Entwicklung nach 28 Tagen: 138 mg CO2/g
- Reißdehnung: 28/38 % (DIN 53455):

### Beispiel 5

75 g (0,35 Mol) einer Hydroxypropylcellulose mit MS 0,93 wird in 1000 g Propionsäure suspendiert, mit 1,5 ml coc. Schwefelsäure versetzt und 1 h bei 50°C gerührt. Nach sukzessiver Zugabe von 91 g (0,70 Mol) Propionsäureanhydrid und 81 g (0,35 Mol) Nonenylbemsteinsäureanhydrid rührt man das Reaktionsgemisch weitere 4 h bei 50°C, kühlt ab und verdampft nach Zugabe von 5 ml 50proz. NaOH das Lösungsmittel. Der isolierte Thermoplast schmilzt bei 80-90°C.

### Beispiel 6

Man suspendiert 100 g (0,62 Mol) Stärke in 1000 g Dimethylsulfoxid, gibt 5 g DBU zu und erhitzt 1h auf 60°C. Nach Zugabe von 100 g (0,38 Mol) 2-Dodecenylbernsteinsäureanhydrid wird die Temperatur wird für 4 h auf 80°C erhöht und das Produkt anschließend aus Wasser gefällt. Der resultierende Stärkeester schmilzt bei 70-80°C.

### Beispiel 7

Eine Suspension aus 100 g (0,45 Mol) Hydroxypropylguar (MS ca.1) in 900 g Dimethylacetamid, die 1,5 g Kaliumcarbonat enthält wird 1 h bei 60°C gerührt, mit 175 g (0,51 Mol) Octadecenylbernsteinsäureanhydrid versetzt und 4 h auf 100°C erhitzt. Fällung aus Wasser liefert ein Derivat, das bei 130-140°C mechanisch verformbar wird.

## Patentansprüche

1. Thermoplastische und biologisch abbaubare Polysaccharidester/Polysaccharidetherester, die dadurch gekennzeichnet sind, daß sie sich durch die allgemeine Struktur 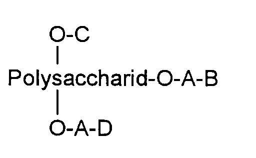 beschreiben lassen, wobei Polysaccharid-O die substituierten OH-Gruppen einer polymeren Saccharideinheit repräsentiert und in der A eine lineare Polyetherkette folgender Struktur ist
A = (E-O)n
in der E eine lineare aliphatische oder aromatische verzweigte oder unverzweigte Kette mit 2 bis 11 C-Atomen bedeutet, n eine ganze Zahl gleich oder größer als 0 ist und B sowie D ein Maleinsäureadditionsprodukt der folgenden Struktur ist 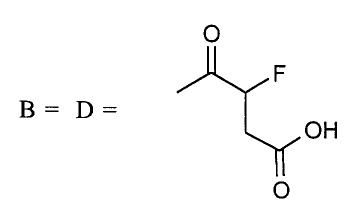 in der F ein aliphatisches, gesättigtes, ein- oder mehrfach ungesättigtes Kohlenstoffgerüst ist, das gegebenenfalls mit weiteren Substituenten versehen sein kann und wobei C ein Wasserstoff, ein oder mehrere Substituenten aus der Gruppe Hydroxyethyl, Hydroxypropyl, Hydroxybutyl, Methyl, Ethyl, Benzyl, Dihydroxypropyl, Carboxyalkyl, Sulfoalkyl oder Cyanoethyl sein kann.

2. Thermoplastische und biologisch abbaubare Polysaccharidester/-etherester gemäß Anspruch 1, dadurch gekennzeichnet, daß sie pro Mol Polysaccharideinheit 0 bis 6,5 Mol Alkylether und 0,1 bis 3 Mol Maleinsäureadditionsprodukt enthalten.

3. Thermoplastische und biologisch abbaubare Polysaccharidester/-etherester gemäß Anspruch 1 oder 2, mit dem Unterschied, daß sich D durch die Struktur 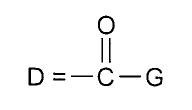 beschreiben läßt, in der G ein aliphatisches C-1 bis C-3 Kohlenstoffgerüst ist, das gegebenenfalls mit weiteren Substituenten versehen sein kann oder 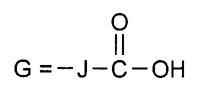 wobei J ein aromatisches oder aliphatisches Kohlenstoffgerüst ist, das gegebenenfalls mit weiteren Substitutienten versehen sein kann.

4. Thermoplastische und biologisch abbaubare Polysacharidester/-etherester gemäß Anspruch 1 bis 4, dadurch gekennzeichnet, daß sie pro Mol Polysaccharideinheit 0 bis 6,5 Mol Alkylether und X Mol Maleinsäureadditionsprodukt sowie Y Mol einer Monocarbonsäure oder einer weiteren Dicarbonsäure enthalten, wobei gilt: 0,1 < X < 2,9, 0,1 < Y < 2,9 und X + Y < 3.

5. Thermoplastische und biologisch abbaubare Polysaccharidester/-etherester gemäß Anspruch 1 bis 4, dadurch gekennzeichnet, daß die Maleinsäureadditionsproduktgruppen B und D, Alkan- und/oder Alkenylbernsteinsäuregruppen mit aliphatischen C-8 bis C-18 Seitenketten sind, die gegebenenfalls mit weiteren Substituenten versehen sein können.

6. Thermoplastische und biologisch abbaubare Polysaccharidester/-etherester gemäß Anspruch 1 bis 5, dadurch gekennzeichnet, daß die Maleinsäureadditionsproduktgruppen B Alkan- und/oder Alkenylbernsteinsäuregruppen mit aliphatischen C-8 bis C-18 Seitenketten sind, die gegebenenfalls mit weiteren Substituenten versehen sein können, die Monocarbonsäurekomponente Acetat und die Dicarbonsäurekomponente Phthalat ist.

7. Verfahren zur Herstellung thermoplastischer und biologisch abbaubarer Polysaccharidester/-etherester gemäß Anspruch 1 bis 6, dadurch gekennzeichnet, daß die Reaktion säurekatalysiert, in einer Monocarbonsäure als Lösungsmittel durchgeführt und das Produkt durch Eindampfen des Reaktionsgemischs isoliert wird.

8. Formteile, Folien, Fasern, Beschichtungen, Blends und Laminate, dadurch gekennzeichnet, das sie mindestens zu 10 % aus den Polysaccharidestern/-etherestern gemäß Anspruch 1 oder 3 bestehen.

9. Verwendung der Materialien gemäß einem der Ansprüche 1 bis 6 als Matrixmaterial für Formulierungen für die verzögerte Freisetzung von Wirkstoffen.

10. Verwendung der Materialien gemäß einem der Ansprüche 1 bis 6 für die Beschichtung von Papier nach dem Extruder-, Dispersions- oder Lackierverfahren sowie als Hotmelts.
